# EUROPEAN PATENT APPLICATION

(11) **EP 1 779 892 A1**
(43) Date of publication of application: **02.05.2007**
(21) Application number: 05766412.0
(22) Date of filing: 20.07.2005
(51) Int. Cl.: A61P 33/02

(54) **ANTIPROTOZOAL AGENT**

(30) Priority: 30.07.2004 JP 2004223485
(71) Applicant: Japan Health Sciences Foundation, Tokyo 103-0001 (JP)
(72) Inventor: FUCHINO, Hiroyuki, c/o Research Center, Tsukuba-shi, Ibaraki 305-0843 (JP); SEKITA, Setsuko, c/o Research Center, Tsukuba-shi, Ibaraki 305-0843 (JP); TAKAHASHI, Marii, c/o Research Center, Tsukuba-shi, Ibaraki 305-0843 (JP); SATAKE, Motoyoshi, c/o Inst. Environmental Science, Tokyo 112-8610 (JP)
(74) Representative: Vossius & Partner
(86) International application number: PCT/JP2005/013268
(87) International publication number: WO 2006/011394

(57) **Abstract**

The present invention provides an antiprotozoal agent, which is particularly preferred as an antileishmanial agent. Specifically, the present invention is directed to an antiprotozoal agent comprising, as an active ingredient, a compound of Formula (I): 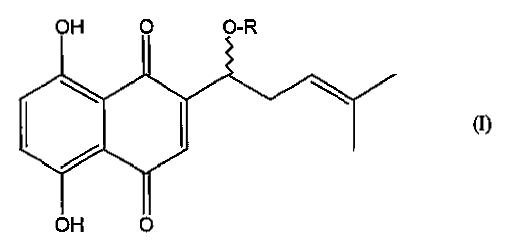 wherein R is -H or acyl,
a salt thereof, or solvate thereof.

## Description

### Technical Field

The present invention relates to an antiprotozoal agent.

### Background Art

In terms of the application of chemotherapy against infectious diseases caused by protozoa, the acquisition of drug resistance by such protozoa is regarded as the most severe problem. For instance, a typical example of such problem is drug resistance in falciparum malaria. Hitherto, some therapeutic agents have been developed. However, protozoa that have acquired resistance to such agents have been found. Thus, protozoal infectious diseases have been spread widely across the world. One such protozoal infectious disease is leishmaniasis.

Leishmaniasis is a parasitic disease that is caused by leishmania protozoa and is endemic to tropical regions, including South America. The disease is one of the six tropical diseases identified by the WHO. There are approximately 12,000,000 patients in total in Africa, the Middle East, Central and South America, and Asia. In addition, 400,000 people are infected with the disease every year. The phlebotomine sandfly, which is a hematophagous insect, is involved in the transmission route. Infection is established when leishmania protozoa in a phlebotomine sandfly invade through bites made when the sandfly sucks blood. The protozoa are parasitic on macrophages, resulting in development of pathological conditions of visceral, cutaneous, and mucocutaneous leishmaniasis. Pathological conditions of visceral leishmaniasis are particularly dangerous, leading to death in severe cases.

Leishmania protozoa are grouped into the following four categories (complexes): *Leishmania donovani; L. tropica; L. Mexicana;* and *L. braziliensi*s. Each complex causes different pathological conditions. Basically, such conditions result from parasitism of protozoa in a particular organ and in local macrophages. Visceral leishmaniasis is caused by parasitism of *L. donovani* in macrophages and reticuloendothelial system cells in the liver, spleen, bone marrow, and the like. Major symptoms thereof involve swelling of the liver and spleen, anemia, leukocytopenia, fever, and lymphadenopathy. Cutaneous leishmaniasis is classified into the following two categories: old world leishmaniasis and new world leishmaniasis. The old world cutaneous leishmaniasis is caused by *L. tropica* and the new world cutaneous leishmaniasis is caused by *L. Mexicana.* Protozoa of each complex are parasitic on skin macrophages so as to form skin ulcers. *L. braziliensis* causes mucocutaneous leishmaniasis by which mucocutaneous and cutaneous lesions are formed. However, it occasionally causes cutaneous leishmaniasis.

As described above, leishmania protozoa are highly-diverse. Also, in view of immunology, there are regional differences in antigenicity, even in cases of similar pathological conditions. This causes difficulties in vaccine development. Thus, there is a substantial need for chemotherapy.

Recently, pentavalent antimony (trade names: Pentostam and Glucantime) has been used as a first-line agent for the treatment of leishmaniasis. In a case in which such agent is not effective, Pentamidine, Amphotericin B, and the like are used. However, these drugs may cause strong adverse drug reactions. Thus, physicians must be careful when using them. In addition, the high cost of antimony has also been problematic.

In addition to the above, Patent Document 1 discloses as therapeutic agents that a germacranolide-type or guaianolide-type sesquiterpenoid compound and a medicament comprising the same. Patent Document 2 discloses a triazole derivative that is effective for the treatment of leishmaniasis.

However, the development of an inexpensive therapeutic agent with few adverse drug reactions has been awaited.

Meanwhile, *"Shiunko"* is a Chinese medicinal ointment that was formulated by the surgeon Seishu Hanaoka in the Edo era. The ointment has been known to have antiinflammatory, analgesic, hemostatic, antiseptic, incarnant, and other effects. It is effective for surgical maladies such as bums, cuts, scratches, hemorrhoids (involving pain, anal fissure, hemorrhage, and anal prolapse caused by hemorrhoids), decubitus ulcers (bed sores), whitlow, and the like. Examples of skin diseases for which the ointment is effective include eczema, housewives' eczema, atopic dermatitis, psoriasis, keratoderma, chapped skin and xerosis (on lips, hands, feet, heels, and elbows), athlete's foot, clavi, callus, verrucae, heat rash, skin irritation, pimples, spots, purulent pustules, boils, pigmented spots, blisters, chilblain, erosion, sores, and insect bites. In particular, the ointment has been demonstrated to be effective for burns since application of the ointment prevents blisters from forming. However, there have been so far no reports of applying *Shiunko* to skin diseases classified as tropical infectious diseases.

*Shiunko* is composed of *Lithospermi Radix* (*shikon*)*, Angelicae Radix* (*tohki*)*,* sesame oil, lard, and beeswax. *Lithospermi Radix* is a root of *Lithospermum erythrorhizon Sieb. Et Zucc (Lithospermum erythrorhizon)* or *Lithospermum euchroma Royle (Macrotomia euchroma (Royle) Pauls),* each of which is a plant belonging to the *Boraginaceae* family. A product derived from the former is referred to as "*ko-shikon.*" A product derived from the latter is referred to as "*nan-shikon*." It has been reported that the components thereof are naphthoquinone dyes such as shikonin, β,β-dimethylacrylshikonin, acetylshikonin, isobutyrylshikonin, isovalerylshikonin, teracrylshikonin, deoxyshikonin, and anhydroalkanine. *Angelicae Radix* to be used is *Angelica acutiloba Kitagawa (ohbuka-tohki)* or *Angelica acutiloba Kitagawa var. sugiyamae Hikino (hokkai-tohki).* It has been reported that the components thereof are butylidenephthalide, safrole, sedanonic acid, valerophenon-o-carboxylic acid, falcarindiol, falcarinol, falcarinolone, and the like.
[Patent Document 1] JP Patent Publication (Kokai) No. 2001-226369 A
[Patent Document 2] JP Patent Publication (Kokai) No. 2003 - 146877 A

### Disclosure of the Invention

It is an objective of the present invention to provide an inexpensive antiprotozoal agent with few adverse drug reactions that is particularly effective as an antileishmanial agent.

The present invention encompasses the following inventions.
(1) An antiprotozoal agent comprising, as an active ingredient, a compound of Formula (I): 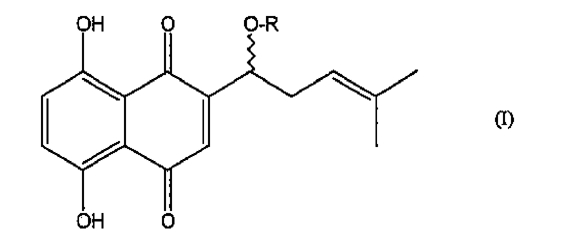 wherein R is -H or acyl,
   a salt thereof, or a solvate of either thereof.
(2) The antiprotozoal agent described in (1), wherein the compound of Formula (I) is a compound of Formula (II): 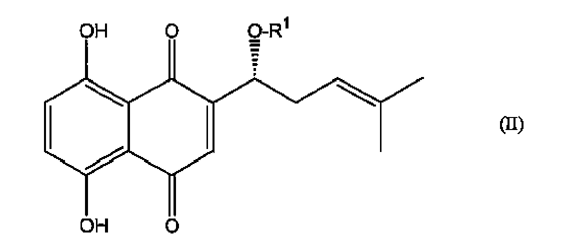 wherein R¹ is -H, -C(=O)CH₃, -C(=O)CH(CH₃)₂, -C(=O)CH₂C(CH₃)₂OH, - C(=O)CH₂CH(CH₃)₂, -C(=O)CH(CH₃)CH₂CH₃ or -C(=O)CH=C(CH₃)₂, or a compound of Formula (III): 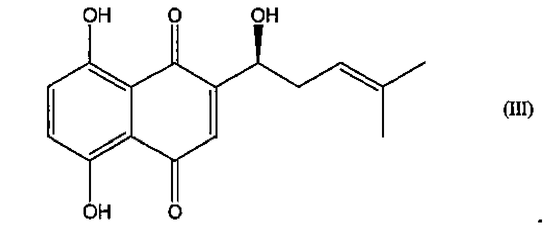
(3) An antiprotozoal agent comprising, as an active ingredient, a substance being derived from *Lithospermi Radix* and having antiprotozoal activity.
(4) An antiprotozoal agent comprising, as an active ingredient, an extract from *Lithospermi Radix* or a treated product thereof.
(5) The antiprotozoal agent described in (3) or (4) comprising, as an active ingredient, an extract from *Angelicae Radix* or a treated product thereof.
(6) An antiprotozoal agent comprising, as an active ingredient, *Shiunko.*
(7) The antiprotozoal agent described in any one of 1 to 6, wherein the agent is an antileishmanial agent.
(8) The antiprotozoal agent according to claim 7, for use in treating leishmaniasis.

In accordance with the present invention, an antiprotozoal agent is provided. The antiprotozoal agent of the present invention is particularly effective for the treatment of leishmaniasis.

The antiprotozoal agent provided by the present invention comprises, as an active ingredient, a naturally-derived substance. Thus, such agent has few problems in terms of adverse drug reactions.

This description includes part or all of the contents as disclosed in the description of Japanese Patent Application No. 2004-223485, which is a priority document of the present application.

### Best Mode for Carrying Out the Invention

The present inventors have found that a strong antiprotozoal effect is imparted to a compound of Formula (1): 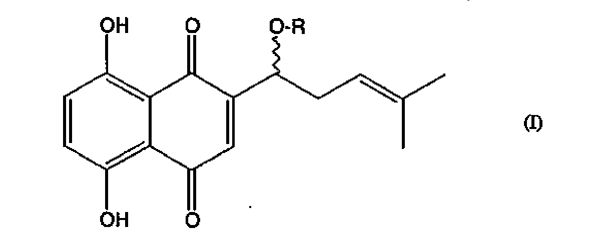 wherein R represents -H or acyl. The compound of Formula (I) has asymmetric carbon atoms. However, the compound may be in the form of optically active substance or raceme. Also, the compound of Formula (I) may be in the form of a salt. In addition, the compound of Formula (I) or a salt thereof may be in the form of a solvate. When the aforementioned salt or solvate is used as an antiprotozoal agent, it must be pharmaceutically acceptable.

When R in Formula (I) is acyl, R may be aliphatic or aromatic acyl. In general, such acyl has 2 to 24 carbon atoms.

A typical example of aliphatic acyl is in the form of linear or branched alkyl having 1 to 23, preferably 1 to 10, more preferably 1 to 8, further preferably 1 to 6, and the most preferably 1 to 4 carbon atoms; linear or branched alkenyl having 2 to 23, preferably 2 to 10, more preferably 2 to 8, further preferably 2 to 6, and the most preferably 2 to 4 carbon atoms; or linear or branched alkynyl having 2 to 23, preferably 2 to 10, more preferably 2 to 8, further preferably 2 to 6, and the most preferably 2 to 4 carbon atoms; to the terminal of which carbonyl is bound to. The aliphatic acyl described above may be substituted with an adequate substituent such as hydroxy, alkoxy having 1 to 6 carbon atoms, thiol, or alkylthio having 1 to 6 carbon atoms. Particularly preferable examples of aliphatic acyl are -C(=O)CH₃, -C(=O)CH(CH₃)₂, - C(=O)CH₂C(CH₃)₂OH, -C(=O)CH₂CH(CH₃)₂, -C(=O)CH(CH₃)CH₂CH₃, and - C(=O)CH=C(CH₃)₂.

A typical example of aromatic acyl is in the form of aryl having 5 to 12 carbon atoms, which may be substituted with an adequate substituent such as hydroxy, alkoxy having 1 to 6 carbon atoms, thiol, or alkylthio having 1 to 6 carbon atoms, and to the terminal of which carbonyl or a group that is induced by removing a single hydrogen atom from the aliphatic acyl described above is bound. Examples thereof include benzoyl, 1-naphthylcarbonyl, and 2-naphthylcarbonyl.

Among the compounds described above, a particularly preferable compound is the following known compound; that is to say, a compound of Formula (II): 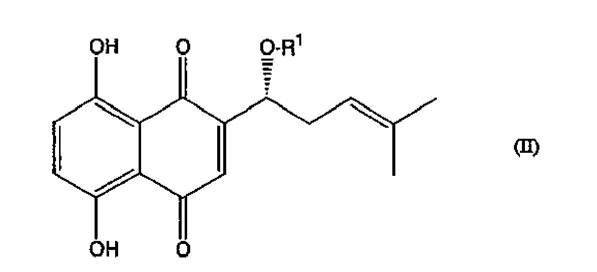 wherein R¹ represents -H (shikonin), -C(=O)CH₃ (acetylshikonin), -C(=O)CH(CH₃)₂ (isobutyrylshikonin), -C(=O)CH₂C(CH₃)₂OH (β-hydroxyisovalerylshikonin), - C(=O)CH₂CH(CH₃)₂ (isovalerylshikonin), -C(=O)CH(CH₃)CH₂CH₃(α-methyl-n-butyrylshikonin), or -C(=O)CH=C(CH₃)₂ (β,β-dimethylacrylshikonin)), or a compound of Formula (III): 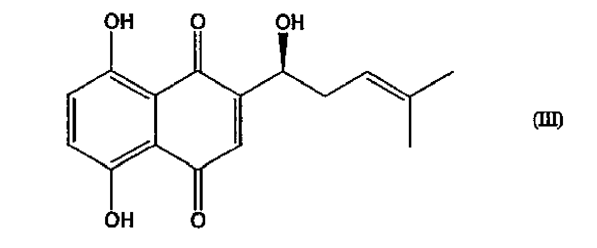 (alkanine).
Also, a mixture of two or more of such compounds can be preferably used.

These known compounds may be chemically synthesized or they may be isolated from a natural product. In addition, commercially available products can be used. For instance, 7 types of compounds based on Formula (II) can be isolated from *Lithospermi Radix* so as to be used. The compound of Formula (III) may be isolated from *Alkanna tinctoria, Onosma echo ides, Lithospermum arvense,* or the like so as to be used. In addition, the compound of Formula (I) may be induced from a natural product. For instance, such compound can be produced from shikonin in accordance with methods described in Bioorganic & Medicinal Chemistry Letters, 12 (10), 1375-1378, 2002 and JP Patent Publication (Kokai) No. 61-151151 A 1986 (date of publication: July 9, 1986).

The present invention also relates to an antiprotozoal agent comprising, as an active ingredient, a substance being derived from *Lithospermi Radix* and having antiprotozoal activity. Further, the antiprotozoal agent of the present invention preferably comprises, as an active ingredient, an extract from *Angelicae Radix* or a treated product thereof.

*Lithospermi Radix* or *Angelicae Radix* used in the present invention is not particularly limited in terms of production area or breed variety. *Lithospermi Radix* or *Angelicae Radix* may be used after being subjected to powderization treatment via disruption, pulverization, or the like. It is preferably used in the form of an extract or a treated product thereof.

As an extraction solvent, hexane is preferable. However, methanol, ethanol, butanol, isopropanol, acetic acid ethyl ester, acetone, benzene, cyclohexane, chloroform, dichloromethane, or the like may also be used. In general, 3 to 10 L of such extraction solvent is used for 1 kg of *Lithospermi Radix* or *Angelicae Radix.*

There is no particular limitation in terms of extraction temperature. The extraction temperature may be between the melting point and the boiling point of a solvent. Supercritical extraction may also be carried out. In addition, extraction is usually carried out under atmospheric pressure; however, it may be carried out under pressurization or depressurization. Extraction time differs depending on extraction temperature and the like. In general, it is 6 to 24 hours.

An extract obtained as described above is filtrated using cloth, a stainless filter, a filter paper, or the like such that impurities and the like are removed therefrom. Thus, an extract of interest can be obtained. In addition, an extract may be subjected to treatments such as spray dry treatment, freeze-dry treatment, and super critical treatment after being filtrated.

An extract obtained as above can be directly used as an active ingredient of the antiprotozoal agent of the present invention. In addition, such extract may be used as a treated product having improved activity by treating it using a variety of purification means such as ion exchange chromatography, gel filtration chromatography, and dialysis.

In addition, the antiprotozoal agent of the present invention can be formulated by combining the aforementioned compound, a salt thereof, a solvate of either thereof, or an extract from or a treated product of *Lithospermi Radix* or *Angelicae Radix* with a known carrier for a medicament. The form of administration is not particularly limited, and thus it may be adequately selected according to need. In general, the antiprotozoal agent is used in the forms of oral agents such as tablets, capsules, granules, fine grains, powders, liquids, syrups, suspensions, emulsions, and elixirs. Alternatively, it is used in the forms of parenteral agents such as parenteral injections, infusions, suppositories, inhalants, transdermal therapeutic systems, transmucosal therapeutic systems, adhesive preparations, and ointments.

The dose of the antiprotozoal agent of the present invention differs depending upon patient's age and weight, upon the severity of the malady, and upon the route of administration. In the case of oral administration, the dose thereof is 10 to 3000 mg per day of, for example, dried powder of a hexane extract from *Lithospermi Radix.* Also, in the case of oral administration, the doses are generally administered 1 to 3 times per day.

The oral agent of the present invention is manufactured using excipients such as starch, lactose, saccharose, mannitol, carboxymethylcellulose, corn starch, and inorganic salts in accordance with a conventional method.

For this type of formulation, in addition to the aforementioned excipients, binders, disintegrating agents, surfactants, lubricants, fluidity-promoting agents, flavoring agents, coloring agents, perfumes, and the like can be adequately used.

Specific examples of binders include crystalline cellulose, crystalline cellulose carmellose sodium, methylcellulose, hydroxypropylcellulose, low substituted hydroxypropylcellulose, hydroxypropylmethylcellulose, hydroxypropylmethylcellulosephthalate, hydroxypropylmethylcellulose acetate succinate, carmellose sodium, ethylcellulose, carboxymethylethylcellulose, hydroxyethylcellulose, wheat starch, rice starch, corn starch, potato starch, dextrin, pregelatinized starch, partially-pregelatinized starch, hydroxypropyl starch, pullulan, polyvinyl pyrrolidone, aminoalkyl methacrylate copolymer E, aminoalkyl methacrylate copolymer RS, methacrylate copolymer L, methacrylate copolymer, polyvinylacetal diethylaminoacetate, polyvinyl alcohol, gum arabic, gum arabic powder, agar, gelatin, white shellac, traganth, refined white sugar, and macrogol.

Specific examples of disintegrating agents include crystalline cellulose, methylcellulose, low substituted hydroxypropylcellulose, carmellose, carmellose calcium, carmellose sodium, croscarmellose sodium, wheat starch, rice starch, corn starch, potato starch, partially-pregelatinized starch, hydroxypropyl starch, sodium carboxymethyl starch, and traganth.

Examples of surfactants include soybean lecithin, sucrose fatty ester, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate, and lauromacrogol.

Examples of lubricants include wheat starch, rice starch, corn starch, stearic acid, calcium stearate, magnesium stearate, hydrous silicon dioxide, light silicic anhydride, synthetic aluminium silicate, dried aluminum hydroxide gel, talc, magnesium metasilicate aluminate, calcium hydrogen phosphate, anhydrous dibasic calcium phosphate, sucrose fatty ester, waxes, hydrogenated vegetable oil, and polyethylene glycol.

Specific examples of fluidity-promoting agents include hydrous silicon dioxide, light silicic anhydride, dried aluminum hydroxide gel, synthetic aluminium silicate, and magnesium silicate.

In addition, the antiprotozoal agent used in the present invention may comprise flavoring and coloring agents when being administered in the forms of liquids, syrups, suspensions, emulsions, and elixirs.

The antiprotozoal agent of the present invention may be used in the forms of so-called foods for specified health uses (e.g., antileishmanial foods) and the like when added to foods, chewing gum, beverages, and the like.

*Shiunko,* which is an existing Chinese medicine comprising an ingredient derived from *Lithospermi Radix* and *Angelicae Radix,* is also preferable as the antiprotozoal agent of the present invention. Since *Shiunko* has been known as an ointment that is significantly effective for skin injuries, it can be applied in the case of leishmaniasis, particularly in cases such as cutaneous leishmaniasis or mucocutaneous leishmaniasis. Examples of *Shiunko* that can be used include commercially available products provided by Tsumura & Co., Maruishi Pharmaceutical Co., Ltd., Kanebo Pharmaceutical Co., Ltd., and Matsuura Kanpo Co., Ltd. In addition, *Shiunko* can be produced in accordance with a known method as described below. First, sesame oil is heated. Beeswax is added thereto so as to become melted therein. Next, lard is added thereto so that all ingredients are allowed to become completely melted therein. *Angelicae Radix* is added thereto. After the surface color of *Angelicae Radix* becomes a burnt color and sufficient extraction is achieved, *Angelicae Radix* is removed therefrom and *Lithospermi Radix* is added thereto. After sufficient extraction is achieved, *Lithospermi Radix* is removed therefrom. The resultant is filtrated such that any remaining residue is removed. The resulting product is allowed to stand so as to become solidified. The solid product is sufficiently kneaded using an ointment slab so that it may then be used.

The present invention is hereafter described in greater detail with reference to the following examples, although the technical scope of the present invention is not limited thereto.

### Examples

### [Example 1]

A hexane extract from *Lithospermi Radix* (obtained by the method used for Reference example 1), a hexane extract from a mixture of *Lithospermi Radix* and *Angelicae Radix* (obtained by the method used for Reference example 2), shikonin (Nagara Science), acetylshikonin (Nagara Science), β,β-dimethylacrylshikonin (Nagara Science), isobutyrylshikonin (Nagara Science), α-methyl-n-butyrylshikonin (Nagara Science), isovalerylshikonin (Nagara Science), β-hydroxyisovalerylshikonin (Nagara Science), and alkanine (Wako Pure Chemical Industries, Ltd.) were subjected to antileishmanial activity measurement.

First, 1 to 5 mg of each sample was dissolved in dimethylsulfoxide (DMSO) such that DMSO solutions (10 mg/ml) were prepared. Then, the solutions were diluted with Medium 199 (800 µg/ml) and allowed to pass through a membrane filter. The resulting test solutions were adjusted to 9 different concentrations. The test solutions (50 µl) at different concentrations and a leishmania culture solution (50 µl) that had been adjusted to a final concentration of 2 × 10⁵ /ml were introduced into wells of a microtiter plate such that the total volume of each culture solution was 100 µl in each well. After incubation at 27°C in the presence of 5%CO₂ for 48 hours, 10 µl of a Tetracolor ONE reagent was added thereto, followed by incubation for 6 hours. Thereafter, the OD value (450-630 nm) was measured using a microplate reader. Each sample at each concentration (3 wells) was subjected to measurement such that the mean value was obtained. Then, a graph was created such that the IC₅₀ value was obtained. Table 1 shows the results.

**[Table 1]**

| | IC₅₀ (µg/ml) |
|---|---|
| Hexane extract from *Lithospermi Radix* | 0.17 |
| Hexane extract from a mixture of *Lithospermi Radix* and *Angelicae Radix* | 0.20 |
| Shikonin | 0.09 |
| Acetylshikonin | 0.026 |
| β,β-dimethylacrylshikonin | 0.014 |
| Isobutyrylshikonin | 0.017 |
| α-methyl-n-butyrylshikonin | 0.014 |
| Isovalerylshikonin | 0.02 |
| β-hydroxyisovalerylshikonin | 0.19 |
| Alkanine | 0.028 |

As is apparent from table 1, the hexane extract from *Lithospermi Radix,* the hexane extract from a mixture of *Lithospermi Radix* and *Angelicae,* and shikonin-related compounds were all shown to have strong antileishmanial activity.

### (Reference example 1)

### Preparation of a hexane extract from Lithospermi Radix

Dried *Lithospermi Radix* that had been finely pulverized using a mill was placed in an eggplant-type flask. Hexane at a volume 30 times that of the pulverized *Lithospermi Radix* was added thereto, followed by extraction at 45°C for 5 hours. Then, filtration was carried out. The resulting filtrate was subjected to concentration and dehydration such that the extract thereof was obtained.

### (Reference example2)

### Preparation of a hexane extract from a mixture of Lithospermi Radix and Angelicae Radix

Equivalent amounts of dried *Lithospermi Radix* and dried *Angelicae Radix* that had been finely pulverized using a mill were weighed and placed in a eggplant-type flask. Hexane at a volume 30 times that of the mixture of *Lithospermi Radix* and *Angelicae Radix* was added thereto, followed by extraction at 45°C for 5 hours. Then, filtration was carried out. The resulting filtrate was subjected to concentration and dehydration such that an extract thereof was obtained.

### [Example 2]

*Shiunko* was administered to patients that were affected with cutaneous leishmaniasis such that treatment effects of *Shiunko* were confirmed *in vivo.*

The *Shiunko* used was *Shiunko* produced by Matsuura Kanpo Co., Ltd. A formulation af this *Shiunko* (1 g) was obtained by mixing, as starting materials, 0.08 g of *Angelicae Radix,* 0.30 g of beeswax, 0.12 g of *Lithospermi Radix,* 0.03 g of lard, and 1.0 g of sesame oil, transferring the active ingredients of the crude drugs *(Angelicae Radix* and *Lithospermi Radix)* to an oil phase (i.e., carrying out extraction), and removing the residues of the crude drugs (extraction residues).

The test subjects were 26 cutaneous leishmaniasis patients. An age- and sex-specific breakdown of the patients is given below.

**[Table 2]**

| Age- and sex-specific breakdown of test subjects | | | |
|---|---|---|---|
| Age | Male | Female | Total |
| 0-5 | 2 (7.69%) | 1 (3.83%) | 3 (11.53%) |
| 6-10 | 0 | 1 (3.83%) | 1 (3.83%) |
| 11-20 | 4 (15.38%) | 2 (7.69%) | 6 (23%) |
| 21-29 | 5 (19.23%) | 0 | 5 (19.23%) |
| 30-39 | 3 (11.53%) | 4 (15.38%) | 7 (26.92%) |
| 40-49 | 3 (11.53%) | 1 (3.83%) | 4 (15.38%) |
| Total | 17 (65.38%) | 9 (34.61%) | 26 |

The above 26 test subjects were instructed to apply an adequate amount of *Shiunko* twice daily for 4 weeks. In addition, they were examined by a physician in charge of the clinical trial once a week.

Clinical observation concerning appearance in terms of sizes of lesions (mm), conditions of the lesions (dry or wet), and the like were carried out before, during, and after treatment. Responses to the treatment were evaluated based on a four-stage classification.

Final results for the follow-up examination of 14 out of 26 test subjects (53.84%) were obtained. Table 3 shows the results. *Shiunko* was confirmed to be effective for the treatment of cutaneous leishmaniasis.

**[Table 3]**

| Classification | Evaluation | (Response) | Number of relevant test subjects (% of 14 subjects) |
|---|---|---|---|
| 1 | Significantly effective | (Almost completely recovered or cured) | 3 (21.42%) |
| 2 | Sufficiently effective | (Obviously recovered) | 8 (57.14%) |
| 3 | Effective | (Slightly recovered) | 2 (14.28%) |
| 4 | Not effective | (Not recovered or remaining unchanged) | 1 (7.14%) |
| Total | | | 14 |

All publications, patents, and patent applications cited herein are incorporated herein by reference in their entirety.

## Claims

1. An antiprotozoal agent comprising, as an active ingredient, a compound of Formula (I): 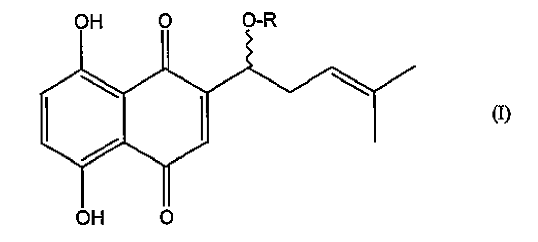 wherein R is -H or acyl,
a salt thereof, or a solvate of either thereof.

2. The antiprotozoal agent according to claim 1, wherein the compound of Formula (I) is a compound of Formula (II): 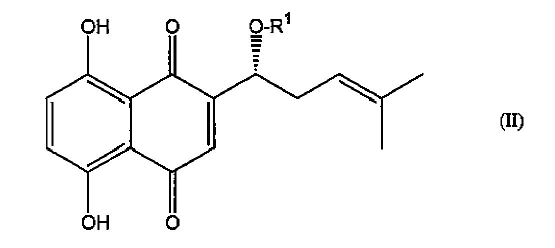 wherein R¹ is -H, -C(=O)CH₃, -C(=O)CH(CH₃)₂, -C(=O)CH₂C(CH₃)₂OH, - C(=O)CH₂CH(CH₃)₂, -C(=O)CH(CH₃)CH₂CH₃ or -C(=O)CH=C(CH₃)₂, or
a compound of Formula (III):

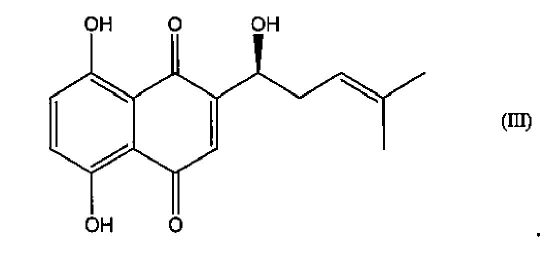

3. An antiprotozoal agent comprising, as an active ingredient, a substance being derived from *Lithospermi Radix* and having antiprotozoal activity.

4. An antiprotozoal agent comprising, as an active ingredient, an extract from *Lithospermi Radix* or a treated product thereof.

5. The antiprotozoal agent according to claim 3 or 4 comprising, as an active ingredient, an extract from *Angelicae Radix* or a treated product thereof.

6. An antiprotozoal agent comprising, as an active ingredient, *Shiunko.*

7. The antiprotozoal agent according to claim 1, 2, 3, 4, or 6, wherein the agent is an antileishmanial agent.

8. The antiprotozoal agent according to claim 7, for use in treating leishmaniasis.
